# EUROPÄISCHE PATENTSCHRIFT

(11) **EP 1 691 827 B1**
(45) Veröffentlichungstag und Bekanntmachung des Hinweises auf die Patenterteilung: **19.08.2009**
(21) Anmeldenummer: 05752350.8
(22) Anmeldetag: 24.06.2005
(51) Int. Cl.: A61K 38/36, A61P 7/00, A61P 9/00

(54) **VERWENDUNG VON PEPTIDEN, DIE AUS DER B BETA KETTE DES HUMANEN FIBRINOGENS ABGELEITET WURDEN, ZUR BEHANDLUNG VON SCHOCK**
USE OF PEPTIDES DERIVED FROM THE B BETA CHAIN OF HUMAN FIBRONOGEN FOR THE TREATMENT OF SHOCK
UTILISATION DES PEPTIDES DERIVES DE LA CHAINE B BETA DU FIBRINOGENE HUMAINE POUR LE TRAITEMENT DU CHOC

(30) Priorität: 25.06.2004 AT 10872004; 13.01.2005 AT 402005
(43) Veröffentlichungstag der Anmeldung: 23.08.2006
(73) Patentinhaber: Fibrex Medical Research & Development GmbH, 1010 Wien (AT)
(72) Erfinder: PETZELBAUER, Peter, A-1230 Wien (AT); ZACHAROWSKI, Kai, 40593 Düsseldorf (DE)
(74) Vertreter: Schwarz, Albin
(86) Internationale Anmeldenummer: PCT/AT2005/000228
(87) Internationale Veröffentlichungsnummer: WO 2006/000007

(56) Entgegenhaltungen:
- WO-A-02/48180
- WO-A-99/02565
- DATABASE BIOSIS [Online] BIOSCIENCES INFORMATION SERVICE, PHILADELPHIA, PA, US; 2003, ZACHAROWSKI, K. ET AL.: "A small molecule derived from fibrinogen, Bbeta15-42, reduces myocardial inflammation and injury via inhibition of the adhesion molecule VE-cadherin" XP002350542 Database accession no. 2004:19507
- KNÖBL, P.: "Pathophysiologie und Therapie von Sepsis-assoziierten Gerinnungsstörungen" WIENER MEDIZINISCHE WOCHENSCHRIFT, Bd. 152, Nr. 21-22, 2002, Seiten 559-563, XP002350540

## Beschreibung

### Hintergrund der Erfindung

Die vorliegende Erfindung ist auf eine pharmazeutische Zubereitung zur Behandlung von Schock gerichtet.

Ein Schock ist eine akute Komplikation vieler verschiedener pathologischer Zustände, die durch die Unfähigkeit des Herz-Kreislauf-Systems, einen ausreichenden Durchblutungsdruck aufrechtzuerhalten, gekennzeichnet ist. Infektionserreger können direkt oder indirekt ein Versagen des Herz-Kreislauf-Systems bewirken. Bakterien, Bakteriengifte, Viren und nicht zuletzt eine unzureichende zelluläre oder humorale Wirtsreaktion, einhergehend mit einer Entzündung und Gerinnung, können zu einem Verlust an Gefäßtonus, einem Verlust an Gefäßbarrierenfunktion, einem Verlust an myokardialer Kontraktionskraft und einem Verlust an Organfunktion führen, was alleine oder in Kombination zu einem Schock und schließlich zum Tod des Patienten führt. Die Behandlung einer bakteriellen Infektion beruht auf einer antibiotischen Behandlung, welche die Bakterien abtötet, jedoch die Toxinämie nicht behandelt und die unzureichende zelluläre oder humorale Reaktion nicht korrigiert. Bei gramnegativen Bakterien ist Lipopolysaccharid (LPS oder Endotoxin) für das Auslösen eines gramnegativen Schocks verantwortlich. Grampositive Bakterien können ein multiples Organversagen und einen septischen Schock ohne Endotoxämie verursachen, die Zellwand von grampositiven Bakterien enthält jedoch ebenfalls Toxine wie Lipoteichonsäure (LTA) und Peptidoglykan (PepG). LTA und PepG wirken in Synergie, um Cytokine, wie z.B. den Tumomekrosefaktor (TNF) α und Interferon (IFN) γ, freizusetzen, und zwar um iNOS zu induzieren und schließlich Schock und Organversagen zu verursachen.

Endotoxämie, Sepsis und septischer Schock stehen mit der Erzeugung großer Mengen von Stickstoffoxid (NO) in Zusammenhang. Die übermäßige Gefäßerweiterung und vaskuläre Hyporeaktivität gegenüber blutdruckerhöhenden Mitteln, welche mit einem Kreislaufschock einhergehen, können mit Inhibitoren der induzierbaren Isoform der NO-Synthase (iNOS) rückgängig gemacht werden (Southan und Szabo, Biochem Pharmacol. 1996;51:383-94, Thiemermann Gen Pharmacol 1997; 29:159-66), iNOS-Inhibitoren reduzieren jedoch nicht die von Toxinen verursachte Organschädigung (Wray et al. Shock 1998;9:329-335).

Die Behandlung eines durch Virusinfektionen verursachten Schocks ist eine sogar noch größere Herausforderung, da für die meisten Infektionen keine Antivirusmittel verfügbar sind. Behandlungen, die allein auf die Beseitigung des Infektionserregers abzielen, sind bei Patienten mit einem Schock aufgrund eines Infektionserregers nicht ausreichend, da vom Infektionserreger ausgelöste sekundäre Vorgänge, welche mit einer Entzündungs-reaktion und Veränderungen des Gerinnungssystems einhergehen, möglicherweise unabhängig wurden und zum Tod des Patienten führen, ungeachtet der Frage, ob der ursächliche Infektionserreger neutralisiert wurde oder nicht. Eine spezifische Behandlung ist nicht verfügbar, und somit zielen derzeitige Verfahren darauf ab, die Symptome zu lindern, was eine mechanische Ventilation, den Ersatz von Flüssigkeit, die Anwendung herzwirksamer Arzneimittel, die strenge Kontrolle der Sauerstoffsättigung, des Hämoglobins, der Glucose und der Nierenfunktion einschließt. Die alleinige Kontrolle der Entzündungsreaktion, z.B. mittels hoch dosierter Steroide, oder die Hemmung der Gerinnung mit Antithrombin bringt keine Verbesserung der Überlebensrate. Das einzige Molekül, bei dem sich bisher erwies, dass es hinsichtlich der Verringerung der Mortalität eine bemerkenswerte Wirksamkeit besitzt, ist das ,aktivierte Protein C', welches mit Koagulation/Fibrinolyse und den Entzündungsprozessen wechselwirkt.

Ein Schock während des Verlaufs einer Infektion hängt zumeist mit offensichtlichen oder nicht offensichtlichen Veränderungen des Plasmafibrinogens zusammen, begleitet von einer Fibrinbildung und einem Anstieg der Fibrinfragmente. Diese Aktivierung von Gerinnung und fibrinolytischen Pfaden kann zu einer offensichtlichen oder nicht offensichtlichen disseminierten intravaskulären Koagulation (DIC) führen, welche einen Gefäßverschluss und Endorganschaden zur Folge hat, und zu einem Verbrauch an Gerinnungsfaktoren, der Blutungen zur Folge hat. Eine Sepsis ist die häufigste Ursache einer DIC. Wichtig ist, dass Fibrinogen, Fibrin und Fibrinfragmente nicht nur bei der Blutgerinnung eine Rolle spielen, sondern mehrere Bindungsstellen für Zell- und Matrixproteine aufweisen, welche ihnen das Wechselwirken mit weißen Blutkörperchen, Blutplättchen, Endothelzellen und Matrixstrukturen ermöglichen. Dies führt zur Zellaktivierung, Zellwanderung, einem Freisetzen von Cytokinen und letztendlich zu einer Entzündungsreaktion. Die Rolle, die Fibrinogen oder Fibrin bei der Entzündung spielt, ist umfassend dokumentiert (besprochen von Altieri Thromb Haemost 82:781-786; Herrick et al. Int J Biochem Cell Biol 31:741-46). Der D-Bereich des Moleküls enthält zahlreiche Bindungsstellen für Matrixmoleküle, Endothelzellen, Blutplättchen und Entzündungszellen. Der E-Bereich des Fibrins bindet an CD11c (Loike et al. Proc Natl Acad Sci USA 88:1044-48).

Vor kurzem beschrieben wir eine neue Rolle für die Bbeta₁₅₋₄₂-Sequenz des Fibrins bei der Entzündung (WO 02/48180). Diese Sequenz ist ebenfalls im E-Bereich des Fibrins lokalisiert und ist nur dann aktiv, wenn Fibrinopeptid gespalten wird. Fibrinfragmente, welche diese Sequenz an ihrem freien N-Terminus der beta-Kette enthalten, binden an das Endothelium und verursachen eine Entzündung, und ein Peptid, das mit den Aminosäuren 15-42 der Bbeta-Kette des Fibrins zusammenpasst, blockiert die Bindung von Fibrin-fragmenten an die Endotheloberflächen und blockiert in vitro die Entzündung (WO 02/48180). In vivo verhindert dieses Peptid eine myokardiale Entzündung und verringert die Ausmaße eines Myocardinfarkts in Situationen der Ischämie / Reperfusion (WO 02/48180).

Fibrinfragmente treten in jedweder Situation mit beeinträchtiger Fibrinbildung und beeinträchtiger Fibrinolyse auf. Besonders in Situationen des Schocks aufgrund eines Infektionserregers stellen diese veränderte Fibrinbildung und diese veränderte Fibrinolyse ein großes Problem dar. Bei zahlreichen Erkrankungen wurde eine direkte Wechselbeziehung zwischen dem Resultat und der Beeinträchtigung der Fibrinbildung / Fibrinolyse dokumentiert. Z.B. Dengue (van Gorp et al. J Med Virol 2002, 67:549-54, Mairuhu et al. Lancet Inf Dis 2003; 3:33-41). In WO 99/02565 ist die Verwendung eines Liganden für Fibrinogen und/oder Fibrin zur Herstellung eines Mittels zur Behandlung und/oder Prophylaxe von Mikrozirkulationsstörungen und/oder zur Beeinflussung der Rheologie eines Säugers geoffenbart. Das Atemnotsyndrom beim Erwachsenen (ARDS) ist eine Form einer akuten Lungenschädigung, die durch eine rötliche extravaskuläre Fibrin-ablagerung gekennzeichnet ist (Idell Am J Respir Med. 2002; 1:383-91). Eine Thrombose in den pulmonalen Gefäßen und eine disseminierte intravaskuläre Koagulation wurden im Zusammenhang mit ARDS ebenfalls beobachtet.

Die Gründe für das Fortbestehen/weltweite Auftreten von Dengue-Fieber (DF) und hämorrhagischem Dengue-Fieber (DHF) als großem Problem der Volksgesundheit sind komplex, Vektorkontrollmaßnahmen waren nicht erfolgreich, um DF/DHF auszumerzen. Derzeit liegt der Hauptschwerpunkt der Finanzierungsmittel am öffentlichen Sektor für die Dengue-Forschung (im Jahr 2001 auf 15 Millionen US$ geschätzt) bei der Molekularepidemiologie, der Immunpathophysiologie, der Forschung zur Entdeckung von Impfstoffen der zweiten Generation und bei neuen oder verbesserten Ansätzen zur Vektorkontrolle.

Mehrere ImpfstoffKandidaten befinden sich in den USA und in Thailand im klinischen Versuchsstadium, am Markt gibt es jedoch noch immer kein Arzneimittel zur Behandlung infizierter Patienten und, was noch schlimmer ist, scheinen derzeit keinerlei kommerziellen Aktivitäten zur Erforschung und Entwicklung einer Chemotherapie im Gange zu sein. Die Weltgesundheitsorganisation veröffentlichte strategische Richtlinien zur Bekämpfung von DF/DHF, welche - als Ziele von hoher Priorität - die Entwicklung von Antivirusmitteln, die auf Protease oder andere kaum untersuchte Enzyme gerichtet sind; die Entwicklung von Antivermittlern, die auf die Ursachen einer erhöhten Gefäßdurchlässigkeit oder geänderten Hämostase gerichtet sind, umfassen.

### Kurzfassung der Erfindung

Die Erfindung betrifft die Verwendung eines Peptids mit der allgemeinen Formel II 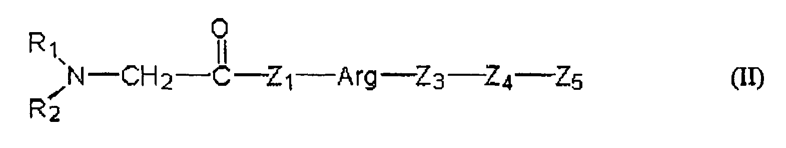 worin
R₁ und R₂ gleich oder unterschiedlich, Wasserstoff, einen gesättigten oder ungesättigten Kohlenwasserstoffrest mit 1 bis 10, insbesondere 1 bis 3 Kohlenstoffatomen bedeuten,
Z₁ einen Histidinrest bedeutet,
Arg einen Argininrest bedeutet,
Z₃ einen Prolin- oder Valinrest bedeutet,
Z₄ einen Leucin- oder Valinrest bedeutet,
Z₅ ein von der Bbeta-Kette des Fibrins abgeleiteter Peptidrest ist,
   welches Peptid die biologische Eigenschaft besitzt, mit dem induzierbaren VE-Cadherin-Bindungsmotiv an der Bβ-Kette (d.h. Bβ₁₅₋₄₂) des menschlichen Fibrins zusammenzupassen, für die Herstellung einer pharmazeutischen Zubereitung zur Behandlung von Schock.
   Überdies wird bevorzugt verwendet ein Peptid, in welchem
Z₅ ein Peptidrest mit der Aminosäuresequenz
Asp Lys Lys Arg Glu Glu Ala Pro Ser Leu Arg Pro Ala Pro Pro Ile Ser Gly Gly Gly Tyr Arg
Z₁ ein Histidinrest,
Arg ein Argininrest,
Z₃ ein Prolinrest, und
Z₄ ein Leucinrest ist.

Die Erfindung betrifft ferner die Verwendung eines Peptids, welches die N-terminale Sequenz 
Gly-His-Arg-Pro-Leu-Asp-Lys-Lys-Arg-Glu-Glu-Ala-Pro-Ser-Leu-Arg-Pro-Ala-
-Pro-Pro-Pro-Ile-Ser-Gly-Gly-Gly-Tyr-Arg
 aufweist und welches die biologische Eigenschaft besitzt, mit dem induzierbaren VE-Cadherin-Bindungsmotiv an der Bβ-Kette (d.h. Bβ₁₅₋₄₂) des menschlichen Fibrins zusammenzupassen, für die Herstellung einer pharmazeutischen Zubereitung zur Behandlung von Schock.
Eine weitere bevorzugte Ausgestaltung der erfindungsgemäßen Verwendung ist dadurch gekennzeichnet, dass das Peptid 
Gly-His-Arg-Pro-Leu-Asp-Lys-Lys-Arg-Glu-Glu-Ala-Pro-Ser-Leu-Arg-Pro-Ala-
-Pro-Pro-Pro-Ile-Ser-Gly-Gly-Gly-Tyr-Arg
 ist.
Es hat sich gezeigt, daß mit den oben genannten Peptiden inbesondere Schockzustände behandelt werden können, wobei der Schock mit einem oder mehreren aus der Gruppe, umfassend Bakteriengifte, disseminierte intravaskuläre Koagulopathie, nektrotisierende Fasciitis, hämorrhagischen Schock infolge einer Virusinfektion, insbesondere verursacht durch Filovirus, Arenaviridae, Bunyaviridae, Flavivirus, Dengue, akutes hämorrhagisches Atmungsversagen, verursacht durch Infektions-erreger oder Autoimmunerkrankungen, Organversagen nach einer Organschädigung, insbesondere durch einen Myocardinfarkt, eine Gefäßoperation, ein Abklemmen von Organen, einen hämorrhagischen Schock, Lungeninfarkt, Leberinfarkt, Darminfarkt, operative Eingriffe und Schlaganfall, und die Organfehlfunktion bei transplantierten Organen, in Zusammenhang steht.

### Detaillierte Beschreibung der Erfindung

### Peptide und Proteine

Peptide wurden durch eine Festphasen-Peptidsynthese hergestellt und mittels einer Umkehrphasen-HPLC gereinigt, wobei Nucleosil 100-10C18-Säulen (PiChem, Graz, Österreich) verwendet wurden. Es sollte angemerkt werden, dass der beta 15-42-Bereich unter Spezien 100% gleichartig ist, wenn konservative Aminosäure-Substitutionen ermöglicht werden. Der aus den Aminosäuren Aα1-51, Bβ1-118 und γ1-78 zusammen-gesetzte N-terminale Disulfidknoten von Fibrinogen (NDSK) wurde wie zuvor beschrieben hergestellt (WO 02/48180). Der aus den Aminosäuren Aα17-51, Bβ15-118 und γ1-78 zusammengesetzte N-terminale Disulfidknoten von Fibrin (NDSK-II, dem die Fibrino-peptide A und B fehlen) wurde hergestellt, indem NDSK bei 37 °C 3 Stunden lang mit Thrombin (20 U /1mg NDSK) behandelt wurde. Das restliche Thrombin wurde bei 37 °C 2 Stunden lang mit 10 mM Diisopropylfluorophosphat (Fluka, Milwaukee, WI) neutralisiert. Alle Produkte wurden daraufhin in eine phosphatgepufferte Salzlösung (PBS) dialysiert.

### ELISA

### Peptid Bβ₁₅₋₄₂ bindet an VE-Cadherin

Die Wechselwirkung der Bbeta-Kette (Bbeta₁₅₋₄₂) von Fibrin mit Endothelzellen verursacht morphologische Veränderungen (Bunce et al. J Clin Invest 89:842-50; Bach et al. Exp Cell Res 238:324-34; Chalupowicz et al. J Cell Biol 130:207-15; Hamaguchi et al. Blood 81:2348-56; Francis et al. Blood cells 19:291-306), eine Proliferation (Sporn et al. Blood 86:1802-10), das Freisetzen von von-Willebrand-Faktor (Ribes et al. J Clin Invest 79:117-23, Ribes et al. J Clin Invest 84: 435-42; Erban und Wagner, J Biol Chem 267, 2451-58) und möglicherweise IL-8 (Qi et al. Blood 90:3593-3602) und eine Membranexpression von CD54 (Harley et al. Art Thromb Vasc Biol 20:652-658). VE-Cadherin wurde als Bindungsligand der Sequenz Bbeta₁₅₋₄₂ identizifiert und ELISAs wurden entwickelt, um diese Wechselwirkung von Endothelzellen und/oder VE-Cadherin mit Fibrin oder Fibrin-fragmenten nachzuweisen. Martinez et al. verwendeten anti-Pan-Cadherin-Antikörper zum Einfangen von Cadherinen aus Endothelzellen, gefolgt von einer Inkubation mit Fibrin (Martinez et al. Ann NY Acad Sci 936:386-405), HUVEC-Monoschichten (welche VE-Cadherin exprimieren) wurden mit radiomarkierten Fibrinfragmenten oder Peptid Bbeta₁₅₋₄₂ überschichtet (Bach et al. J Biol Chem 273:30719-28; Harley et al. Art Thromb Vasc Biol 20:652-658), und von Gorlatov und Medved wurde rekombinantes VE-Cadherin verwendet (Biochemistry 41:4107-16). Andere setzten zum Nachweisen von Fibrinfragmenten im Blut einen ELISA ein, wobei sie hauptsächlich Antikörper gegen verschiedene Sequenzen innerhalb des Fibrinogenmoleküls verwendeten, einschließlich Antikörpern gegen das Bbeta₁₅₋₄₂-Motiv (besprochen bei Fareed et al. Clin Chem 8:1845-53).

Wir entwickelten einen modifizierten ELISA, der mit denselben, von anderen beschriebenen Prinzipien arbeitet, der Zweck des hier beschriebenen ELISA besteht jedoch nicht darin, Fibrin-Abbauprodukte quantitativ zu bestimmen, sondern nach Proteinen, Peptiden oder Verbindungen zu suchen, welche die Bindung der Bbeta₁₅₋₄₂-Sequenz und des VE-Cadherins stören. Das Prinzip besteht darin, dass das VE-Cadherin entweder als verkürztes Protein, als Vollprotein oder gekoppelt mit anderen Proteinen, welche die Bbeta₁₅₄₂-Bindungsstelle nicht stören, mit der Bbeta₁₅₋₄₂-Sequenz von Fibrin wechselwirken darf. Man kann jegliche andere zusätzliche Substanz in dieses System einbringen und messen, ob diese Substanz die VE-Cadherin/Bbeta₁₅₋₄₂-Bindung hemmt.

Im Einzelnen wurden 96-Mulden-Proteinimmobilisierungsplatten (Exiqon, Vedbaek, DK) mit rekombinantem menschlichem VE-Cadherin-FC-Fusionsprotein (8 nM; R&D Systems, Minneapolis) in PBS beschichtet und über Nacht bei 4 °C stehengelassen. Die Platten wurden danach gewaschen und mit Peptid Bβ₁₅₋₄₂ (GHRPLDKKREEAPSL RPAPPPISGGGYR), das am C-Terminus des Peptids mit einer FLAG-Sequenz (DYKDDDDK) markiert war, oder mit einem FLAG-markierten Zufallspeptid (DRGAPAHRPPRGPISGRSTPEKEKLLPG) inkubiert, und zwar in einer Konzentration von 0-80 µMol. Nach dem Waschen wurde durch Inkubation mit einem Peroxidase-markierten anti-FLAG-Antikörper (Sigma, St. Louis, USA) und einem chromogenen Substrat gebundenes FLAG-markiertes Peptid nachgewiesen. Die optische Dichte wurde durch einen auf eine Wellenlänge von 450 nm eingestellten ELISA-Plattenleser bestimmt. Die Daten stellen den Mittelwert aus drei unabhängigen Versuchen dar, wobei jeder dreifach durchgeführt wurde. Die untenstehende Tabelle zeigt, dass das Peptid Bβ₁₅₋₄₂ in konzentrationsabhängiger Weise an VE-Cadherin band. Im Gegensatz dazu zeigte das Zufallspeptid nur eine unwesentliche Bindung.

**Dosisabhängige Bindung von Peptid Bbeta₁₅₋₄₂ an VE-Cadherin**

| | | | | | | | | | | | |
|---|---|---|---|---|---|---|---|---|---|---|---|
| µM | | 0 | 0,23 | 0,7 | 2,3 | 7 | 14 | 21 | 35 | 46 | 70 |
| 15-42 FLAG | Mittelwert | 0 | 0,01 | 0,02 | 0,08 | 0,33 | 0,92 | 1,3 | 1,5 | 1,93 | 2,1 |
| | Standardabweichung | 0 | 0,01 | 0.01 | 0,03 | 0,17 | 0,19 | 0,2 | 0 | 0 | 0 |
| | | | | | | | | | | | |
| Zufalls-FLAG | Mittelwert | 0 | 0,01 | 0 | 0,01 | 0,03 | 0,12 | 0,2 | | 0,35 | 0,5 |
| | Standardabweichung | 0 | 0,01 | 0 | 0,01 | 0,02 | 0,04 | 0,1 | | 0 | 0 |

Das Peptid Bβ₁₅₋₄₂ und Fibrinfragmente konkurrieren hinsichtlich der Bindung an VE-Cadherin.

In einem nächsten Schritt analysierten wir, ob dieser ELISA zum Screenen nach anderen Peptiden/Verbindungen angewandt werden kann, damit diese mit der Bindung der Bβ₁₅₋₄₂-Sequenz an VE-Cadherin konkurrieren. Erwartungsgemäß hemmte das Peptid Bβ₁₅₋₄₂ vollständig die Bindung des flag-markierten Peptids Bβ₁₅₋₄₂ und wurde als positive Kontrolle verwendet, und Zufallspeptide oder ein Lösungsmittel hatten keinerlei Wirkung und wurden als negative Kontrollen verwendet. Kürzere Peptide hemmten teilweise die Bindung von Bβ₁₅₄₂ an VE-Cadherin. NDSK-II hemmte die Bβ₁₅₋₄₂-Bindung in konzentrationsabhängiger Weise. Ein Gleichgewicht zwischen Bβ₁₅₋₄₂ und NDSK-II (50%ige Hemmung) war bei einem Molverhältnis von 24:1 erreicht. NDSK hatte wenig oder keine Wirkung.

Die Kunststoffoberfläche wurde mit VE-Cadherin in einer Konzentration von 8 nM beschichtet. Danach wurden die angegebenen Peptide in Konzentrationen von 200 µM hinzugefügt, NDSK oder NDSK-II wurde in den angegebenen Konzentrationen hinzugefügt. Der Nachweis der Bindung des FLAG-markierten Bbeta₁₅₋₄₂ (12 µM) wurde wie obenstehend beschrieben durchgeführt.

| Blockierendes Reagens | % Hemmung der 15-42FLAG-Bindung an VE-Cadherin |
|---|---|
| | Mittelwert ± Standardabweichung |
| Peptid 15-42 (28mer) | 100 ± 10 |
| Peptid zufällig (4mer) | 3 ± 3 |
| Peptid zufällig (28mer) | 10 ±3 |
| Lösungsmittel | 0 + 0 |
| Peptid 15-18 (4mer) 200 µM | 65 ± 12 |
| Peptid 15-26 (12mer) 200 µM | 64 ± 10 |
| Peptid 15-30 (16mer) 200 µM | 61 ± 13 |
| Peptid 15-34 (20mer) 200 µM | 67 ± 17 |
| Peptid 15-37 (24mer) 200 µM | 17 ± 19 |
| Peptid 16-42 (27mer) 200 µM | 55 ±13 |
| | |
| Peptid 15-18 (4mer) 12 µM | 7 ± 2 |
| Peptid 15-26 (12mer) 12 µM | 6 ± 1 |
| Peptid 15-30 (16mer) 12 µM | 6 ± 3 |
| Peptid 15-34 (20mer) 12 µM | 7 ±1 |
| Peptid 15-37 (24mer) 12 µM | 7± 2 |
| Peptid 16-42 (27mer) 12 µM | 5 ± 2 |
| | |
| NDSK-II 0,06 µM | 1 + 0 |
| NDSK-II 0,12 µM | 39 + 18 |
| NDSK-II 0,20 µM | 42+14 |
| NDSK-II 0,60 µM | 52 + 16 |
| NDSK-II 1,2 µM | 63+13 |
| NDSK-II 2,4 µM | 79+9 |
| NDSK-II 4,0 µM | 82 + 12 |
| NDSK 0,06 µM | 0 + 0 |
| NDSK 0,12 µM | 2 + 1 |
| NDSK 0,20 µM | 1 + 1 |
| NDSK 0,60 µM | 7 + 6 |
| NDSK 1,2 µM | 15 + 13 |
| NDSK 2,4 µM | 16 + 9 |
| NDSK 4,0 µM | 20 + 10 |
| anti-VE-Cadherin Ab (TEA1/31, | 1 mg/ml) 2 + 1 |

Wirksamkeit des Peptids bbeta₁₅₋₄₂ bei der Behandlung von Denguevirus-infizierten Mäusen. Materialien und Methoden.

Virus. Das Denguevirus des Typs 2 (DEN-2), Stamm P23085, wurde von der Staatlichen Virensammlung, Moskau, Russland, in Form einer gefriergetrockneten Suspension von infiziertem ICR-Mäusegehirn erhalten. Das erhaltene Denguevirus wurde im Gehirn der ICR-Mäusejungtieren Passagen unterzogen, so wie zuvor beschrieben (Atrasheuskaya et al. FEMS Immunology and Medical Microbiology. 35, 33-423). Eine 10%ige Gehirnsuspension diente als Virusstamm und wurde bei -40° C gelagert. Der Virustiter wurde durch die serienmäßigen Verdünnungen der Gehirnsuspension bestimmt. Die Gehirnsuspension wurde in Gruppen von jeweils 10 Mäusen (4 Wochen alte BALB/c) i.p. geimpft, und die Mortalität wurde aufgezeichnet. Der Virustiter wurde berechnet und betrug 7,4 1g LD50/ml.
Jegliche Arbeit mit dem infektiösen Virus wurde im Sicherheitsraum der höchsten Biosicherheitsstufe 3 (BSL-3) im Labor des SRC VB «Vector» (Russland) durchgeführt. Tiere.

Vier Wochen alte, durch Inzucht erzeugte männliche BALB/c-Mäuse (Haplotyp H-2d) wurden vom Vivarium des Staatlichen Forschungszentrums für Virologie und Biotechnologie «Vector» erhalten. Die Tiere wurden mit Futter und Wasser, das ad libitum verfügbar war, in einzelne Käfige gegeben.
Analysen.

Den Mäusen wurde vor der Infektion und nach der Exposition an DEN-2 unter Methoxyfluran-Anästhesie aus dem orbitalen Sinus Blut entnommen. Für jeden Zeitpunkt wurden drei Mäuse zur Blutgewinnung herangezogen.

Die zirkulierenden Blutplättchen (PLT), roten Blutkörperchen (RBC), weißen Blutkörperchen (WBC), Hämoglobin (HGB) und Hämatokrit (HCT) wurden unter Verwendung eines Cell -Dyn 900-Hämatologie-Analysators (Sequoia-Turner corporation, USA, CA) bestimmt.
Ein Teil des gewonnenen Blutes wurde zentrifugiert, um Serum zu erhalten, das bis zum Ende des Versuchs bei -80°C gelagert wurde. Die Serumspiegel von Cytokinen wurden gemessen, wobei von R&D Systems (Minneapolis,USA) hergestellte Enzym-Immunoassay-Kits gemäß den Instruktionen des Herstellers verwendet wurden. Die Nachweisgrenzen waren wie folgt: TNF-α, weniger 5,1 pg/ml; Interleukin (IL)-1β, 3,0 pg/ml; IL-6, 3,1 pg/ml; IFNγ- weniger 20 pg/ml.

Das Denguevirus im Blut der Tiere wurde mittels RT-PCR identifiziert, so wie zuvor beschrieben (Harris et al. J. Clin. Microbiol. 36, 2634-2639). Die Gesamt-RNA aus dem Blut wurde unter Verwendung eines Kits von Quiagen (Deutschland) isoliert. Die Primer waren wie folgt: oberer 5'AATATGCTGAAACGCGAGAGAAACCG (Position 136-161), unterer 5'AAGGAACGCCACCAAGGCCATG (Position 237-258), wobei sie ein 119 bp-Produkt ämplifizierten. Um die Virusbelastung quantitativ zu bestimmen, wurde DEN-2 wie zuvor beschrieben auf Vero E6-Zellkulturen titriert (Harris et al. J. Clin. Microbiol. 36, 2634-2639). Am 0. und 22. Tag nach der Exposition wurde das Blut der überlebenden Mäuse mittels ELISA in Hinblick auf anti-DEN-2-Antikörper (IgG) analysiert, so wie zuvor beschrieben (Ignatyev et al. J. Biotechnology. 44, 111-118). Versuchsaufbau.

Durch Inzucht erzeugte, vier Wochen alte männliche BALB/c-Mäuse wurden in 6 Hauptgruppen aufgeteilt. Jede Gruppe enthielt 50 Mäuse. Alle Tiere wurden intraperitoneal (i.p.) mit dem an Mäuse angepassten DEN-2-Stamm P23085 (wie obenstehend beschrieben) in einer Dosierung von 1 LD₅₀ infiziert und täglich auf Anzeichen einer Krankhaftigkeit untersucht. Mäuse aus den ersten Untergruppen aller Hauptgruppen (A1-F1) wurden zur Mortalitätskontrolle herangezogen. Jede Untergruppe enthielt 20 Mäuse. Tiere der zweiten Untergruppen (A2 - F2) wurden zum Erhalten von Serumproben verwendet. Jede Untergruppe enthielt 30 Mäuse.
Gruppenbeschreibung. n= 50 in jeder Gruppe
Die Kontrollgruppe erhielt nur das Virus.

Die Behandlung mit Peptid Bβ₁₅₋₄₂ wurde zweimal täglich mit jeweils 4800 µg/kg durch eine intraperitoneale Injektion durchgeführt, und zwar ab dem 3. Tag nach der Infektion bis zum 8. Tag nach der Infektion.
Blut- und Serumproben wurden an den ausgewählten Zeitpunkten beschafft: am 1.,3., 5., 7., 11., 22. Tag nach der Exposition.
Eine statistische Analyse wurde unter Anwendung des Student's t- or Chi-Quadrat-Tests durchgeführt. P-Werte <0,05 wurden als signifikant betrachtet.

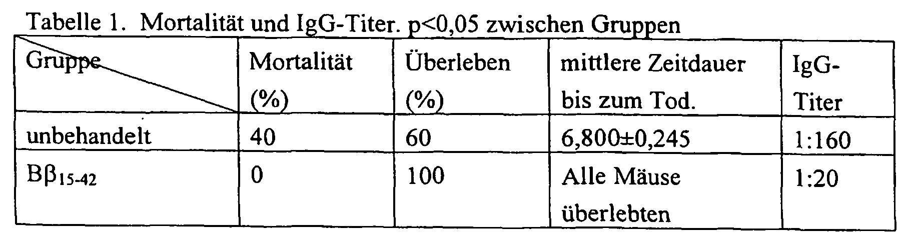

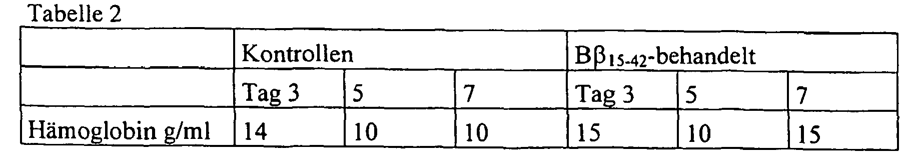

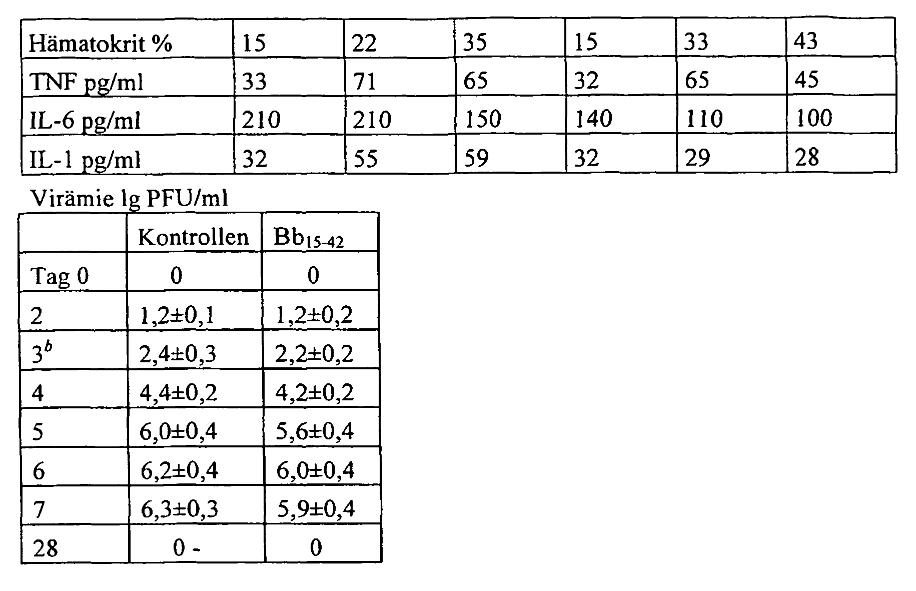

### Gramnegativer Schock

Männliche Wistar-Ratten mit einem Gewicht von 230-280 g waren in der Tierversuchsanlage (Universität Düsseldorf) untergebracht und wurden mit Standardkost und Wasser *ad libitum* gefüttert. Alle Vorgänge wurden gemäß den AAALAC-Richtlinien und dem *Handbuch für die Pflege und Verwendung von Labortieren* (Amt für Gesundheit und Soziales, Nationale Gesundheitsinstitute, Veröffentlichung Nr. 86-23) ausgeführt. Außerdem waren alle Versuche von einer Behörde für Ethik und Forschung der Universität Düsseldorf und des Landes bewilligt. Wie zuvor beschrieben (Zacharowski et al. Crit Care Med 2000, Zacharowski et al. Crit Care Med 2001; 29:1599-1608), wurden die Ratten mit Natriumthiopenton (120 mg/kg i.p.) anästhesiert, und die Anästhesie wurde je nach Bedarf mit ergänzenden Dosierungen von Natriumthiopenton aufrechterhalten.

In die Luftröhre wurde eine Kanüle eingeführt, um die Atmung zu ermöglichen, und die rektale Temperatur wurde mit einer homöothermen Decke bei 37 °C gehalten. Die rechte Halsschlagader wurde katheterisiert und mit einem Druckfühler verbunden, um den phasischen und mittleren Arterienblutdruck (MAP) und die Pulszahl (HR) zu messen, welche auf einem Datenerfassungsystem (MacLab 8e, ADI Instruments, Deutschland) angezeigt wurden, das auf einem IBM-Computer installiert war. In die rechte Drosselvene wurde zur Verabreichung von Arzneimitteln eine Kanüle eingeführt. In die Blase wurde ebenfalls eine Kanüle eingeführt, um den Urinfluss zu ermöglichen und um die Möglichkeit der Entwicklung eines später auftretenden Nierenversagens zu verhindern. Alle Tiere erhielten während des gesamten Versuchs einen vollständigen Flüssigkeitsersatz von 1,0 ml/kg/h (0,9 % Natriumchlorid, Kochsalzlösung, als i.v.-Infusion in die Drosselvene). Nach Beendigung des chirurgischen Eingriffs wurde ermöglicht, dass sich die kardiovaskulären Parameter 15 Minuten lang stabilisierten, und sie wurden 6 Stunden lang ständig aufge-zeichnet. Bei diesem Modell eines LPS-induzierten multiplen Organversagens ist ein Zeitraum von 6 Stunden wesentlich, um einen erheblichen Anstieg der Serumspiegel von AST und ALT zu erzielen, während ein erheblicher Anstieg der Serumspiegel von Harnstoff und Kreatinin bereits nach 2 Stunden beobachtet werden kann.

Drei Gruppen wurden untersucht:
Die Ratten wurden einer Scheinoperation unterzogen: (Simulation).
Die Ratten wurden einem gramnegativen Schock ausgesetzt. Lipopolysaccharid von E. coli, Serotyp 0,127:B8 (6 mg/kg i.v.), wurde 5 Minuten lang i.v. verabreicht, 1 Stunde später erhielten die Tiere eine Kochsalzlösung (2,4 ml/kg): (LPS + Kochsalzlösung).
Die Ratten wurden einem gramnegativen Schock ausgesetzt. Lipopolysaccharid von E. coli, Serotyp 0,127:B8 (6 mg/kg i.v.), wurde 5 Minuten lang i.v. verabreicht, die Tiere erhielten Bβ₁₅₋₄₂ (2,4 mg/kg): (LPS + Bβ₁₅₋₄₂).
Überleben n=20 in jeder Gruppe, p<0,05

| Simulation | LPS plus Kochsalzlösung | LPS plus Bβ₁₅₋₄₂ |
|---|---|---|
| 100 % | 25% | 88% |

Sechs Stunden nach dem Auslösen eines gramnegativen Schocks wurde aus dem in der rechten Halsschlagader platzierten Katheter Blut entnommen. Die Blutprobe wurde zentrifugiert (1610 x g für 3 Minuten bei Raumtemperatur), um Plasma abzutrennen. Die folgenden Markerenzyme wurden im Plasma als biochemische Indikatoren für eine multiple Organschädigung/Fehlfunktion gemessen:
Eine Leberschädigung wurde bewertet, indem der Anstieg der Plasmaspiegel von Alaninaminotransferase (ALT, ein spezifischer Marker für eine Leberparenchymschädigung) and Aspartataminotransferase (AST, ein nicht spezifischer Marker für eine Leber-schädigung) gemessen wurde.
Eine Nierenfehlfunktion wurde abgeschätzt, indem die Anstiege der Plasmaspiegel von Harnstoff (ein Indikator für eine beeinträchtigte Ausscheidungsfunktion der Niere und/oder einen erhöhten Katabolismus) und Kreatinin (ein Indikator für eine reduzierte Glomerulusfiltrationsrate und folglich eine Nierenfehlfunktion) gemessen wurden. Die Plasmaspiegel von Glucose und Amylase wurden als indirekte Marker der Pankreasfunktion und -schädigung gemessen.
Außerdem wurde das arterielle pO₂ als indirekter Marker der Lungenfunktion/-schädigung gemessen. Laborwerte

| | Simulation | Kontrolle | Bβ₁₅₋₄₂ |
|---|---|---|---|
| ALT | 39,8 | 542,3 | 261,9 |
| SEM | 5,2 | 117,2 | 42,7 |
| | | | |
| AST | 194,1 | 908,8 | 529,0 |
| SEM | 30,8 | 140,9 | 75,7 |
| | | | |
| Kreatinin | 0,5 | 0,9 | 0,6 |
| SEM | 0,0 | 0,1 | 0,1 |
| | | | |
| Harnstoff | 49,1 | 123,2 | 107,8 |
| SEM | 5,8 | 4,6 | 5,9 |
| | | | |
| Glucose | 131,5 | 75,3 | 45,8 |
| SEM | 7,5 | 5,4 | 9,1 |
| | | | |
| Amylase | 1713,3 | 1837,4 | 1945,1 |
| SEM | 131,5 | 122,7 | 176,8 |
| | | | |
| pO2 | 90,0 | 67,0 | 98,7 |
| SEM | 1,8 | 3,7 | 8,2 |

### Mittlerer Arteriendruck

| Zeit | Simulation | | LPS | | LPS + | |
|---|---|---|---|---|---|---|
| | | | | | Bβ₁₅₋₄₂ | |
| (h) | Mittelwert | SEM | Mittelwert | SEM | Mittelwert | SEM |
| | | | | | | |
| 0 | 120,9 | 5,5 | 118,9 | 5,4 | 128,9 | 5.2 |
| 1 | 111,5 | 10,6 | 90,7 | 5,5 | 83,7 | 4.6 |
| 2 | 113,2 | 7,3 | 100,2 | 5,1 | 102,3 | 4 |
| 3 | 116,4 | 5,4 | 90 | 7,4 | 103,2 | 4 |
| 4 | 108,7 | 8,9 | 81,1 | 7,9 | 101,2 | 3 |
| 5 | 104,1 | 9,6 | 60,1 | 10,8 | 97,1 | 5,4 |
| 6 | 104,1 | 9,6 | 34,1 | 7,7 | 107,7 | 9,6 |

### Pulszahl

| Zeit | Simulation | | LPS | | LPS | |
|---|---|---|---|---|---|---|
| | | | | | Bβ ₁₅₋₄₂ | |
| (h) | Mittelwert | SEM | Mittelwert | SEM | Mittelwert | SEM |
| | | | | | | |
| 0 | 482,2 | 17 | 457 | 18,1 | 436,9 | 6,4 |
| 1 | 461,7 | 12,1 | 511,2 | 27,4 | 484,8 | 18,6 |
| 2 | 488,1 | 13,6 | 523,3 | 27,3 | 484,1 | 10,3 |
| 3 | 506,6 | 26,6 | 518,5 | 24,9 | 509,8 | 12 |
| 4 | 488,9 | 17,4 | 516,7 | 32,1 | 516,7 | 13,4 |
| 5 | 470,4 | 13,9 | 515,5 | 26,1 | 533,7 | 30,7 |
| 6 | 443,7 | 0,7 | 530,1 | 27,7 | 541,6 | 24,4 |

Am Ende des Versuchs wurden von allen untersuchten Gruppen Organ (Lunge, Leber, Herz und Niere)-Biopsien entnommen. Die Biopsien wurden bei Raumtemperatur in einer gepufferten Formaldehydlösung (4% in phosphatgepufferter Kochsalzlösung) fixiert und nach Wien versandt. Mit Standard-H&E gefärbte Abschnitte zeigten keinerlei Unterschiede. Bei Kontrollen, die nur LPS erhielten, fanden wir jedoch in Abschnitten, die unter Verwendung von saurem Fuchsin-Orange G hinsichtlich Fibrinablagerungen gefärbt wurden, Zahlen von Fibrinthrombi, die im Vergleich zu Tieren, welche mit LPS plus Bβ₁₅₋₄₂ (p<0,05) behandelt wurden, signifikant erhöht waren. In scheinbehandelten Tieren waren keine Fibrinthrombi vorhanden.

**durchschnittliche Zahl von Fibrinthrombi in Gefäßen**

| | LPS plus Kochsalzlösung | LPS plus Bbeta₁₅₋₄₂ |
|---|---|---|
| Herz | 28+13 | 5 + 2 |
| Niere | 17+8 | 2+9 |
| Leber | 47 + 41 | 78 + 37 |
| Lunge | 1,7+1 1 | 1 + 0 |

## Patentansprüche

1. Verwendung eines Peptids mit der allgemeinen Formel II 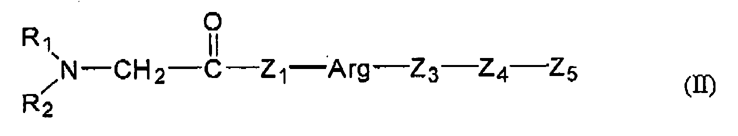 worin
R₁ und R₂ gleich oder unterschiedlich, Wasserstoff, einen gesättigten oder ungesättigte Kohlenwasserstoffrest mit 1 bis 10 Koh lenstoffatomen bedeuten,
Z₁ einen Histidinrest bedeutet,
Arg einen Argininrest bedeutet,
Z₃ einen Prolin- oder Valinrest bedeutet,
Z₄ einen Leucin- oder Valinrest bedeutet,
Z₅ ein von der Bbeta-Kette des Fibrins abgeleiteter Peptidrest ist,
welches Peptid die biologische Eigenschaft besitzt, mit dem induzierbaren VE-Cadherin-Bindungsmotiv an der Bβ-Kette (d.h. Bβ₁₅₋₄₂) des menschlichen Fibrins zusammenzupassen, für die Herstellung einer pharmazeutischen Zubereitung zur Behandlung von Schock.

2. Verwendung nach Anspruch 1, worin der gesättigte oder ungesättigte Kohlenwasserstoffrest in der Bedeutung von R₁ und R₂ 1 bis 3 Kohlenstoffatome aufweist.

3. Verwendung nach einem der Ansprüche 1 oder 2, **dadurch gekennzeichnet, daß** Z₅ ein Peptidrest mit der Aminosäuresequenz 
Asp Lys Lys Arg Glu Glu Ala Pro Ser Leu Arg Pro Ala Pro Pro Pro Ile Ser Gly Gly Gly Tyr
Arg
Z₁ ein Histidinrest,
Arg ein Argininrest,
Z3 ein Prolinrest, und
Z₄ ein Leucinrest ist.

4. Verwendung eines Peptids, welches die N-terminale Sequenz Gly-His-Arg-Pro-Leu-Asp-Lys-Lys-Arg-Glu-Glu-Ala-Pro-Ser-Leu-Arg-Pro-Ala- -Pro-Pro-Pro-Ile-Ser-Gly-Gly-Gly-Tyr-Arg aufweist, welches Peptid die biologische Eigenschaft besitzt, mit dem induzierbaren VE-Cadherin-Bindungsmotiv an der Bβ-Kette (d.h. Bβ₁₅₋₄₂) des menschlichen Fibrins zusammenzupassen, für die Herstellung einer pharmazeutischen Zubereitung zur Behandlung von Schock.

5. Verwendung gemäß Anspruch 4, **dadurch gekennzeichnet, dass** das Peptid 
Gly-His-Arg-Pro-Leu-Asp-Lys-Lys-Arg-Glu-Glu-Ala-Pro-Ser-Leu-Arg-Pro-Ala-
-Pro-Pro-Pro-Ile-Ser-Gly-Gly-Gly-Tyr-Arg
 ist.

6. Verwendung gemäß einem der Ansprüche 1 bis 5, wobei der Schock mit einem oder mehreren aus der Gruppe, umfassend Bakteriengifte, disseminierte intravaskuläre Koagulopathie, nektrotisierende Fasciitis, hämorrhagischen Schock infolge einer Virusinfektion, insbesondere verursacht durch Filovirus, Arenaviridae, Bunyaviridae, Flavivirus, Dengue, akures hämorrhagisches Atmungsversagen, verursacht durch Infektionserreger oder Autoimmunerkrankungen, Organversagen nach einer Organschädigung, insbesondere durch einen Myocardinfarkt, eine Gefäßoperation, ein Abklemmen von Organen, einen hämorrhagischen Schock, Lungeninfarkt, Leberinfarkt, Darminfarkt, operative Eingriffe und Schlaganfall, und die Organfehlfunktion bei transplantierten Organen, in Zusammenhang steht.

## Claims

1. The use of a peptide of general Formula II 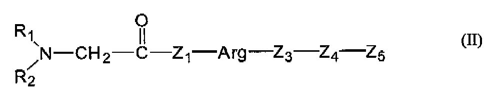 wherein
R₁ and R₂, being equal or different, denote hydrogen, a saturated or unsaturated hydrocarbon moiety comprising from 1 to 10 carbon atoms,
Z₁ denotes a histidine moiety,
Arg denotes an arginine moiety,
Z₃ denotes a proline or valine moiety,
Z₄ denotes a leucine or valine moiety,
Z₅ is a peptide moiety derived from the Bbeta-chain of the fibrin,
which peptide has the biological property of matching the inducible VE-cadherin binding motif on the Bβ-chain (i.e. Bβ₁₅₋₄₂) of human fibrin, for the preparation of a pharmaceutical preparation for the treatment of shock.

2. The use according to claim 1, wherein the saturated or unsaturated hydrocarbon moiety in the meaning of R₁ and R₂ comprises from I to 3 carbon atoms.

3. The use according to any of claims 1 or 2, **characterized in that** Z₅ is a peptide moiety comprising the amino acid sequence 
Asp Lys Lys Arg Glu Glu Ala Pro Ser Leu Arg Pro Ala Pro Pro Pro Ile Ser Gly Gly Gly Tyr
Arg
Z₁ is a histidine moiety,
Arg is an arginine moiety,
Z3 is a proline moiety, and
Z₄ is a leucine moiety.

4. The use of a peptide which exhibits the N-terminal sequence 
Gly-His-Arg-Pro-Leu-Asp-Lys-Lys-Arg-Glu-Glu-Ala-Pro-Ser-Leu-Arg-Pro-Ala-
-Pro-Pro-Pro-Ile-Ser-Gly-Gly-Gly-Tyr-Arg
 which peptide has the biological property of matching the inducible VE-cadherin binding motif on the Bβ-chain (i.e. Bβ₁₅₋₄₂) of human fibrin, for the preparation of a pharmaceutical preparation for the treatment of shock.

5. The use according to claim 4, **characterized in that** the peptide is

Gly-His-Arg-Pro-Leu-Asp-Lys-Lys-Arg-Glu-Glu-Ala-Pro-Ser-Leu-Arg-Pro-Ala-
-Pro-Pro-Pro-Ile-Ser-Gly-Gly-Gly-Tyr-Arg.

6. The use according to any of claims 1 to 5, wherein shock is associated with one or more from the group comprising bacterial toxins, disseminated intravascular coagulopathy, necrotizing fasciitis, haemorrhagic shock following viral infection, in particular caused by filovirus, arenaviridae, bunyaviridae, flavivirus, dengue, acute hemorrhagic respiratory failure caused by infectious agents or autoimmune diseases, organ failure after organ injury, in particular myocardial infarction, vascular surgery, clamping of organs, haemorrhagic shock, lung infarction, liver infarction, gut infarction, surgical procedures and stroke, and organ dysfunction of grafted organs.

## Revendications

1. Utilisation d'un peptide de formule générale II 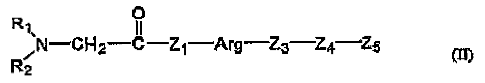 dans laquelle
R₁ et R₂, identiques ou différents, représentent un atome d'hydrogène, un radical hydrocarboné saturé ou insaturé ayant de 1 à 10 atomes de carbone,
Z₁ représente un radical histidine,
Arg représente un radical arginine,
Z₃ représente un radical proline ou valine,
Z₄ représente un radical leucine ou valine,
Z₅ est un radical peptidique dérivé de la chaîne B bêta de la fibrine, lequel peptide possède la propriété biologique d'apparier la chaîne B bêta (à savoir B β₁₅₋₄₂) de la fibrine humaine au motif de liaison VE-cadhérine inductible, pour la production d'une préparation pharmaceutique destinée au traitement du choc.

2. Utilisation selon la revendication 1, dans laquelle le radical hydrocarboné saturé ou insaturé dans la signification de R₁ et R₂, présente 1 à 3 3 atomes de carbone.

3. Utilisation selon l'une quelconque des revendications 1 ou 2, **caractérisée** à en ce que
Z₅ est un radical peptidique avec la séquence d'acides aminés 
Asp Lys Lys Arg Glu Glu Ala Pro Ser Leu Arg Pro Ala Pro Pro
Pro Ile Ser Gly Gly Gly Tyr Arg
Z₁ est un radical histidine,
Arg est un radical arginine,
Z₃ est un radical proline, et
Z₄ est un radical leucine.

4. Utilisation d'un peptide qui présente la séquence N-terminale 
Gly-His-Arg-Pro-Leu-Asp-Lys-Lys-Arg-Glu-Glu-Ala-Pro-
Ser-Leu-Arg-Pro-Ala-Pro-Pro-Pro-Ile-Ser-Gly-Gly-Gly-
Tyr-Arg,
 lequel peptide possède la propriété biologique d'apparier la chaîne B bêta (à savoir B β₁₅₋₄₂) de la fibrine humaine au motif de liaison VE-cadhérine inductible, pour la production d'une préparation pharmaceutique destinée au traitement du choc.

5. Utilisation selon la revendication 4,
**caractérisée en ce que** le peptide est
Gly-His-Arg-Pro-Leu-Asp-Lys-Lys-Arg-Glu-Glu-Ala-Pro-Ser-Leu-Arg-Pro-Ala-Pro-Pro-Pro-Ile-Ser-Gly-Gly-Gly-Tyr-Arg.

6. Utilisation selon l'une quelconque des revendications 1 à 5, dans laquelle le choc est en relation avec l'un ou plusieurs du groupe comprenant les poisons bactériens, la coagulopathie intravasculaire disséminée, la fasciite nécrosante, le choc hémorragique à la suite d'une infection virale en particulier provoquée par les filovirus, les *Arenaviridae*, les *bunyaviridae*, les flavivirus, la dengue, l'insuffisance respiratoire hémorragique aiguë, provoquée par des agents infectieux ou des maladies auto-immunes, une insuffisance d'organe après une lésion organique, en particulier par un infarctus du myocarde, une intervention vasculaire, un sectionnement d'organes, un choc hémorragique, un infarctus pulmonaire, un infarctus hépatique, un infarctus intestinal, des interventions opératoires et un accident cérébro-vasculaire, et le dysfonctionnement d'organes dans le cas d'organes transplantés.
